# EUROPÄISCHE PATENTSCHRIFT

(11) **EP 2 825 250 B1**
(45) Veröffentlichungstag und Bekanntmachung des Hinweises auf die Patenterteilung: **18.01.2023**
(21) Anmeldenummer: 13709773.9
(22) Anmeldetag: 11.03.2013
(51) Int. Cl.: A61M 39/22, A61M 39/24, A61M 1/16, A61M 5/168, A61M 39/10

(54) **SCHLAUCHADAPTER ZUM BEEINFLUSSEN DES DRUCKS INNERHALB EINES SCHLAUCHABSCHNITTS WÄHREND EINER MEDIZINISCHEN BEHANDLUNG**
HOSE ADAPTER FOR INFLUENCING THE PRESSURE INSIDE A HOSE SECTION DURING A MEDICAL TREATMENT
ADAPTATEUR DE TUBULURE SERVANT À INFLUENCER LA PRESSION À L'INTÉRIEUR D'UN TRONÇON DE TUBULURE PENDANT UN TRAITEMENT MÉDICAL

(30) Priorität: 12.03.2012 DE 102012004673; 12.03.2012 US 201261609387 P
(43) Veröffentlichungstag der Anmeldung: 21.01.2015
(73) Patentinhaber: Fresenius Medical Care Deutschland GmbH, 61352 Bad Homburg (DE)
(72) Erfinder: KLEWINGHAUS, Juergen, 61440 Oberursel (DE)
(74) Vertreter: Bobbert & Partner Patentanwälte PartmbB
(86) Internationale Anmeldenummer: PCT/EP2013/000714
(87) Internationale Veröffentlichungsnummer: WO 2013/135365

(56) Entgegenhaltungen:
- WO-A1-00/57935
- WO-A1-96/05883
- WO-A1-96/25214
- WO-A1-98/39589
- WO-A2-01/91829
- DE-B3- 10 319 197
- US-A1- 2009 010 627
- US-A1- 2011 315 611

## Beschreibung

Die vorliegende Erfindung betrifft einen Dialysierflüssigkeitsschlauch gemäß Anspruch 1 sowie eine medizinische Vorrichtung gemäß Anspruch 4.

Aus der Praxis der Dialysebehandlung sind Vorrichtungen bekannt, mittels welcher Dialysierflüssigkeit erwärmt wird, bevor diese in den Dialysator oder Blutbehandlungsfilter, in welchem ein Stoffaustausch über eine - üblicherweise semipermeable - Membran zwischen Blut und Dialysierflüssigkeit erfolgt, eingebracht wird. Manche dieser Heizvorrichtungen erfordern ein sicheres Anliegen oder das Einhalten eines Höchstabstandes zwischen der Heizvorrichtung, welche beispielsweise die Form von Heizwendeln hat, und einem von dieser Heizvorrichtung umgebenen Behältnis, welches beispielsweise als Beutel ausgeführt ist, in welchem sich die zu erwärmende Dialysierflüssigkeit befindet. Bei einer Heizvorrichtung dieser Bauart hängt das Erzielen der gewünschten Erwärmung der Dialysierflüssigkeit davon ab, wie gut das Behältnis an der Heizvorrichtung, beispielsweise ausgeführt als Beutelheizung, anliegt (oder umgekehrt).

Aus der WO 98/39589 A1 sind eine Ventilapparatur und Verfahren hierzu bekannt.

In der WO 01/91829 A2 ist eine Prime-Vorrichtung für medizinische Infusionssysteme offenbart.

Aus der DE 103 19 197 B3 ist eine Dialyse-Anordnung mit federelastischem Ventil bekannt.

Eine Aufgabe der vorliegenden Erfindung ist es, Vorrichtungen und Verfahren vorzuschlagen zum Erzielen oder Sicherstellen eines gewünschten oder erforderlichen Anliegens der Heizvorrichtung an dem die Dialysierflüssigkeit führenden Gefäß oder Behältnis, oder zum Verhindern eines Überschreitens eines maximalen Abstandes zwischen Heizvorrichtung und Gefäß im Gebrauch oder bei der Behandlung des Patienten.

Die erfindungsgemäße Aufgabe wird durch einen Dialysierflüsssigkeitsschlauch mit einem Schlauchadapter mit den Merkmalen des Anspruchs 1 sowie durch eine medizinische Vorrichtung mit den Merkmalen des Anspruchs 4 gelöst.

Erfindungsgemäß wird somit ein Dialysierflüssigkeitsschlauch mit einem Schlauchadapter zum Verbinden mit wenigstens einem Schlauchabschnitt eines Dialysierflüssigkeits-Schlauchsystems vorgeschlagen. Alternativ kann der Schlauchadapter zwischen zwei Schlauchabschnitten eines Dialysierflüssigkeits-Schlauchsystems angeordnet werden oder hierzu vorgesehen und vorbereitet sein. Dabei sind sowohl der Schlauchadapter als auch der Schlauchabschnitt dafür vorgesehen und ausgestaltet, bei ihrem bestimmungsgemäßen Gebrauch von Dialysierflüssigkeit durchströmt zu werden.

Der Schlauchadapter des Dialysierflüssigkeitsschlauchs weist erfindungsgemäß wenigstens einen Verschlussmechanismus zum reversiblen oder vorübergehenden Verschließen eines durchströmbaren Lumens des Schlauchadapters oder des hiermit verbundenen Schlauchabschnitts, und/oder eine Vorrichtung zum Erzeugen oder Verändern eines Strömungswiderstands auf.

Das hierin offenbarte Dialysierflüssigkeits-Schlauchsystem weist wenigstens einen Schlauchadapter auf und/oder ist integral mit einem solchen Schlauchadapter hergestellt oder weist wenigstens einen Verschlussmechanismus zum Verschließen seines durchströmbaren Lumens und/oder wenigstens eine Vorrichtung zum Erzeugen oder Verändern eines Strömungswiderstands auf.

Die medizinische Vorrichtung gemäß der vorliegenden Erfindung weist wenigstens einen erfindungsgemäßen Dialysierflüssigkeitsschlauch auf oder ist hiermit verbunden.

Das hierin offenbarte Verwenden eines Schlauchadapters, wie hierin ebenfalls offenbart, umfasst das Verbinden des Schlauchadapters mit einem Dialysierflüssigkeits-Schlauchsystem, insbesondere mit einer oder mehreren Dialysatorkupplungen hiervon, die zur direkten Verbindung des Dialysierflüssigkeits-Schlauchsystem mit dem Dialysator oder Blutfilter ausgestaltet sind.

Offenbart wird weiterhin ein Verfahren zum Verhindern des Auftretens eines unterhalb eines Mindestdrucks liegenden Drucks in einem Dialysierflüssigkeits-Schlauchsystem. Das Verfahren umfasst dabei entweder das Verbinden eines Schlauchadapters mit dem Dialysierflüssigkeits-Schlauchsystem oder das Anordnen eines Verschlussmechanismus zum Verschließen eines durchströmbaren Lumens des Dialysierflüssigkeits-Schlauchsystems und/oder das Anordnen einer Vorrichtung zum Erzeugen oder Verändern eines Strömungswiderstands innerhalb des Dialysierflüssigkeits-Schlauchsystems. Das Verbinden oder das Anordnen erfolgt dabei stromab einer mit dem Dialysierflüssigkeits-Schlauchsystem verbundenen Heizvorrichtung.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.

Vorteilhafte Weiterentwicklungen der vorliegenden Erfindung sind jeweils Gegenstand von Unteransprüchen und Ausführungsformen.

Erfindungsgemäße Ausführungsformen können eines oder mehrere der im Folgenden genannten Merkmale aufweisen.

In einigen Ausführungsformen der vorliegenden Erfindung ist die Veränderung des Strömungswiderstandes sprungartig, und/oder aber nicht stetig.

In manchen Ausführungsformen der vorliegenden Erfindung stehen der Schlauchadapter und der Schlauchabschnitt des Dialysierflüssigkeits-Schlauchsystems, mit welchem er verbunden ist, in Fluidkommunikation. Der Schlauchadapter wird daher bei seinem Gebrauch von derselben Flüssigkeit und/oder stets derselben Flüssigkeitsmenge durchströmt wie der Schlauchabschnitt.

In einigen Ausführungsformen der vorliegenden Erfindung wird der Strömungswiderstand mittels des Schlauchadapters oder eines seiner Bauteile erzeugt und/oder verändert.

Der Schlauchadapter ist in bestimmten erfindungsgemäßen Ausführungsformen des Dialysierflüssigkeitsschlauchs vorgesehen und/oder ausgestaltet, um zu seinem Gebrauch mit wenigstens einem Schlauchabschnitt eines Dialysierflüssigkeits-Schlauchsystems verbunden zu werden.

In bestimmten Ausführungsformen der vorliegenden Erfindung erfolgt die Veränderung des Strömungswiderstandes innerhalb des durchströmbaren Lumens des Schlauchadapters.

In besonderen Ausführungsformen der Erfindung entspricht die Veränderung des Strömungswiderstandes einem Druckabfall oder bewirkt einen solchen.

In einigen Ausführungsformen der vorliegenden Erfindung verhindert der Verschlussmechanismus in seinem geschlossenen Zustand ein Durchströmen des Schlauchadapters.

In manchen Ausführungsformen der vorliegenden Erfindung verhindert der Verschlussmechanismus in seinem geschlossenen Zustand ein Eintreten von Dialysierflüssigkeit aus einer Quelle für Dialysierflüssigkeit in den Dialysator hinein.

In einigen erfindungsgemäßen Ausführungsformen wird ein Durchströmen des Schlauchadapters infolge des Verschlussmechanismus in Abhängigkeit eines Druckzustands verhindert, der stromauf des Schlauchadapters, und dort insbesondere stromab einer Heizeinrichtung, etwa in Gestalt einer Beutelheizung, herrscht, mittels welcher die durch den Schlauchadapter geführte Dialysierflüssigkeit erwärmt worden ist.

In manchen erfindungsgemäßen Ausführungsformen verändert oder verhindert die Vorrichtung zum Erzeugen eines Strömungswiderstandes beim bestimmungsgemäßen Gebrauch des Schlauchadapters des erfindungsgemäßen Dialysierflüssigkeitsschlauchs eine oder jegliche Fluidströmung stromab des Schlauchadapters und/oder stromauf des Schlauchadapters in Abhängigkeit eines Druckzustands, der stromauf des Schlauchadapters herrscht.

In bestimmten Ausführungsformen der vorliegenden Erfindung dienen der Verschlussmechanismus oder die Vorrichtung zum Erzeugen eines Strömungswiderstands dazu, vorbestimmte Druckzustände stromauf des Schlauchadapters auszuschließen. Hierbei handelt es sich insbesondere um Unterdruckzustände oder Druckzustände unterhalb eines vorbestimmten Mindestdrucks.

Ein Bestimmen oder ein Festlegen des vorbestimmten Mindestdrucks kann durch die Auswahl der einzelnen Elemente wie eines Rückschlagventils oder Druckbegrenzungsventils des Schlauchadapters erfolgen.

Die Begriffe Rückschlagventil oder Druckbegrenzungsventil werden in einigen erfindungsgemäßen Ausführungsformen austauschbar verwendet, wo immer der Fachmann dies als technisch sinnvoll erkennt.

In einigen erfindungsgemäßen Ausführungsformen ist der Mindestdruck ein beliebiger Unterdruck. In bestimmten erfindungsgemäßen Ausführungsformen ist der Mindestdruck ein Druck, bei welchem ein Gefäß, ein Behälter oder ein Beutel der konkret verwendeten Beutelheizung zuverlässig nicht kollabiert, wenn dieser Druck im Inneren hiervon herrscht. In gewissen erfindungsgemäßen Ausführungsformen ist der Mindestdruck ein Druck oder ein Differenzdruck von jeweils 0 hPa.

In einigen erfindungsgemäßen Ausführungsformen bezieht sich der Mindestdruck auf einen Innendruck des Gefäßes, Behälters oder Beutels der konkret verwendeten Beutelheizung; in anderen erfindungsgemäßen Ausführungsformen bezieht sich der Mindestdruck auf einen Druck, welcher im Dialysierflüssigkeits-Schlauchsystem zwischen Heizvorrichtung und Dialysator herrscht.

In bestimmten erfindungsgemäßen Ausführungsformen liegen der Schlauchadapter oder Komponenten hiervon stromaufwärts zum Eingang des Dialysators für Dialysierflüssigkeit.

In einigen erfindungsgemäßen Ausführungsformen strömt in den Schlauchadapter oder Komponenten hiervon dasselbe Fluid ein wie es hieraus auch wieder ausströmt. Eine Bearbeitung des Fluids oder eine Veränderung seiner Zusammensetzung erfolgt in diesen erfindungsgemäßen Ausführungsformen innerhalb des Schlauchadapters nicht. Eine Zugabe von Infusionen oder dergleichen erfolgt in diesen Ausführungsformen ebenfalls nicht innerhalb des Schlauchadapters. Der Schlauchadapter ist hierfür nicht ausgestaltet oder vorgesehen. Der Schlauchadapter ist in bestimmten Ausführungsformen kein Wegeventil oder Mehrwegeventil.

In bestimmten erfindungsgemäßen Ausführungsformen sind der Schlauchadapter oder Komponenten nicht ausgestaltet oder im bestimmungsgemäßen Gebrauch nicht in der Lage, das durchströmende Fluid zu verändern, beispielsweise durch, insbesondere gezieltes oder beabsichtigtes, Erwärmen.

In manchen erfindungsgemäßen Ausführungsformen liegen der Schlauchadapter oder Komponenten hiervon nicht in einer Bypassleitung für die Pumpe.

In gewissen Aspekten der Offenbarung weist das Dialysierflüssigkeits-Schlauchsystem keine Bypassleitung für die Pumpe auf.

In einigen erfindungsgemäßen Ausführungsformen sind der Schlauchadapter oder seine Komponenten keine Vorrichtungen zum Begrenzen eines Drucks oder wirken nicht als solche.

In bestimmten erfindungsgemäßen Ausführungsformen sind der Schlauchadapter oder seine Komponenten Vorrichtungen zum Sicherstellen eines Mindestdrucks oder wirken als solche.

So ist beispielsweise ein hierin genanntes Rückschlagventil oder Druckbegrenzungsventil in einigen erfindungsgemäßen Ausführungsformen nicht vorgesehen, um einen Druck zu begrenzen, indem es bei ausreichend hohem Druck öffnet, sondern es schließt vielmehr, wenn der Druck zu niedrig ist.

Der erfindungsgemäße Dialysierflüssigkeitsschlauch weist in bestimmten Ausführungsformen einen Schlauchabschnitt auf, welcher in eine Pumpe, beispielsweise eine okkludierende Schlauchpumpe wie etwa eine Rollenpumpe, einzulegen ist.

In manchen erfindungsgemäßen Ausführungsformen ist der Schlauchadapter, sein Verschlussmechanismus oder seine Vorrichtung zum Erzeugen oder Verändern eines Strömungswiderstandes ein Ventil und/oder eine Drossel und/oder eine Blende, oder weist wenigstens eines der vorgenannten Elemente auf.

Im Falle eines Ventils kann es beispielsweise ein Rückschlagventil oder Druckbegrenzungsventil mit einem definierten oder vorbestimmten Öffnungsdruck sein.

In bestimmten erfindungsgemäßen Ausführungsformen beträgt der Öffnungsdruck des Verschlussmechanismus des Schlauchadapters mindestens 50 hPa (oder mbar) und/oder maximal 350 hPa.

Der o. g. Mindestdruck beträgt in manchen erfindungsgemäßen Ausführungsformen 5 hPa oder liegt 5 hPa über einem stromab des Schlauchadapters oder des Verschlussmechanismus herrschenden Druck.

In einigen erfindungsgemäßen Ausführungsformen bewirkt die Vorrichtung zum Erzeugen oder Verändern eines Strömungswiderstandes eine Veränderung derart, dass im bestimmungsgemäßen Gebrauch des Schlauchadapters über der Vorrichtung oder über dem Schlauchadapter ein Druckunterschied von mindestens 5 hPa und/oder maximal 1000 hPa, bevorzugt von mindestens 50 hPa und/oder maximal 400 hPa, besonders bevorzugt von mindestens 100 hPa und/oder 350 hPa herrscht.

In manchen Aspekten der Offenbarung ist das Dialysierflüssigkeits-Schlauchsystem integral oder einstückig mit dem Schlauchadapter hergestellt oder fest - beispielsweise nur unter Zerstörung von diesem lösbar - mit diesem verbunden.

In bestimmten Aspekten der Offenbarung weist das Dialysierflüssigkeits-Schlauchsystem zusätzlich einen Behälter (beispielsweise einen Sammel- oder einen Vorratsbehälter) mit Dialysierflüssigkeit auf oder ist hiermit verbunden. Dieses Fluid ist zum Durchströmen des Schlauchsystems vorgesehen.

Zudem kann das Dialysierflüssigkeits-Schlauchsystem optional eine Pumpe aufweisen oder kann mit einer Pumpe verbunden sein, die so angeordnet ist, dass sie die Dialysierflüssigkeit im Lumen des Schlauchsystems fördern kann. Die Pumpe kann als Verdrängerpumpe, beispielsweise als Rollenpumpe ausgestaltet sein.

Bei dem Dialysierflüssigkeits-Schlauchsystem kann es sich in einigen Aspekten der Offenbarung ungeachtet seiner Bezeichnung hierin auch um ein Schlauchsystem für andere medizinische Fluide als Dialysierflüssigkeit handeln, wo immer dies für den Fachmann erkennbar sinnvoll ist.

Der Behälter kann als ein oder mehrere Beutel ausgestaltet sein. Solch ein Behälter findet sich beispielweise in der US 2005/020959 A1.

In einigen Aspekten der Offenbarung weist das Dialysierflüssigkeits-Schlauchsystem eine Heizvorrichtung zum Erwärmen der Dialysierflüssigkeit auf oder ist hiermit verbunden oder weist einen Abschnitt auf, welcher vorgesehen ist, im Gebrauch mit einer Heizvorrichtung verbunden zu werden.

Hierbei ist die Heizvorrichtung in gewissen erfindungsgemäßen Ausführungsformen stromauf des Schlauchadapters positioniert.

Dabei kann die Heizvorrichtung von jenem Typ sein, welcher eines gleichbleibenden, positiven, im Inneren des Behälters herrschenden Drucks bedarf, um die gewünschten Heizergebnisse zu erzielen.

In einigen erfindungsgemäßen Ausführungsformen ist die Heizvorrichtung eine Beutelheizung.

In manchen Aspekten der Offenbarung ist das Dialysierflüssigkeits-Schlauchsystem als Einwegprodukt ausgestaltet.

In einigen erfindungsgemäßen Ausführungsformen ist das offenbarte Dialysierflüssigkeits-Schlauchsystem ein erfindungsgemäßer Dialysierflüssigkeitsschlauch.

In bestimmten erfindungsgemäßen Ausführungsformen der medizinischen Vorrichtung ist diese als Blutreinigungseinrichtung ausgestaltet, beispielsweise als Dialysiervorrichtung, als Filtrationsvorrichtung, als Diafiltrationsvorrichtung oder als Dialysevorrichtung in jedem anderen, dem Fachmann bekannten Sinne.

In gewissen erfindungsgemäßen Ausführungsformen ist die erfindungsgemäße medizinische Vorrichtung ein Behandlungssystem oder eine Behandlungsvorrichtung, mit einem extrakorporalen Blutkreislauf und/oder einem Schlauchsystem, oder sie ist hiermit verbunden.

Die medizinische Vorrichtung ist in bestimmten erfindungsgemäßen Ausführungsformen eine Dialysiervorrichtung, die im Besonderen zur Anwendung für die kontinuierliche veno-venöse Hämodiafiltration (CVV-HDF) und/oder zur Anwendung für die Akutdialyse konfiguriert ist.

In einigen offenbarten Ausgestaltungen des Verwendens des Schlauchadapters wird der Schlauchadapter mit dem Dialysierflüssigkeits-Schlauchsystem stromab einer Heizvorrichtung, insbesondere einer Beutelheizung, verbunden. Dabei ist die Heizvorrichtung ebenfalls mit dem Dialysierflüssigkeits-Schlauchsystem verbunden.

In bestimmten offenbarten Ausgestaltungen des Verwendens des Schlauchadapters wird der Schlauchadapter mit den Dialysatorkupplungen des Dialysierflüssigkeits-Schlauchsystems verbunden. Der Schlauchadapter des erfindungsgemäßen Dialysierflüssigkeitsschlauchs kann hierzu entsprechend ausgestaltet sein, um beispielsweise mit dem oder den Dialysatorkupplungen per Steckverbindung, per Steck-Schraubverbindung oder dergleichen verbunden zu werden, insbesondere ohne Zuhilfenahme von Werkzeug oder weiteren Verbindungselementen.

Unter dem Begriff Schlauch"adapter", wie hierin verwendet, ist nicht etwa einschränkend zu verstehen, dass der Schlauchadapter des erfindungsgemäßen Dialysierflüssigkeitsschlauchs etwas miteinander verbindbar machen soll, was nicht auch ohne ihn verbunden werden kann. Der Begriff umfasst vielmehr auch ein Zwischenelement oder Verbindungselement, welches verwendet werden soll, um zwei Schlauchabschnitte eines Schlauchsystems oder einen Schlauchabschnitt mit einem Dialysator in Fluidkommunikation miteinander, insbesondere unmittelbar, zu verbinden.

In bestimmten Aspekten betrifft die Offenbarung ein Dialysierflüssigkeits-Schlauchsystem, mit wenigstens einem Schlauchadapter zum Verbinden mit wenigstens einem weiteren Schlauchabschnitt des Dialysierflüssigkeits-Schlauchsystems, wobei der Schlauchadapter wenigstens einen Verschlussmechanismus zum Verschließen seines durchströmbaren Lumens und/oder eine Vorrichtung zum Erzeugen oder Verändern eines Strömungswiderstands des Schlauchadapters, des Schlauchabschnitts oder des Dialysierflüssigkeits-Schlauchsystems aufweist, wobei das Dialysierflüssigkeits-Schlauchsystem weiterhin wenigstens einen Behälter mit Dialysierflüssigkeit, welche zum Durchströmen des Schlauchsystems vorgesehen ist, und eine Pumpe, angeordnet zum Fördern der Dialysierflüssigkeit in dem Dialysierflüssigkeits-Schlauchsystem, aufweist oder hiermit jeweils verbunden oder zur Verbindung hiermit vorgesehen ist; wobei das Dialysierflüssigkeits-Schlauchsystem weiterhin eine Heizvorrichtung zum Erwärmen der Dialysierflüssigkeit aufweist oder hiermit verbunden ist oder einen Abschnitt aufweist, welcher vorgesehen ist, im Gebrauch mit einer Heizvorrichtung verbunden zu werden; wobei die Heizvorrichtung im Gebrauch des Dialysierflüssigkeits-Schlauchsystems stromab der Pumpe angeordnet ist, wobei der Schlauchadapter stromab der Heizvorrichtung aber stromauf eines Dialysators angeordnet ist.

Manche oder alle erfindungsgemäßen Ausführungsformen können einen, mehrere oder alle der oben und/oder im Folgenden genannten Vorteile aufweisen.

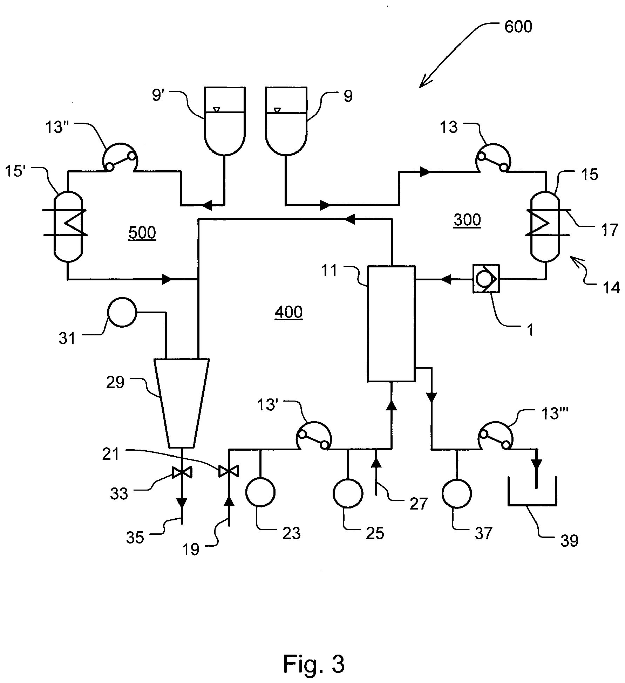

Eine alternative Anordnung im Dialysierflüssigkeits-Schlauchsystem wäre eine Pumpe, beispielsweise eine okkludierende Pumpe (z. B. eine Rollenpumpe), stromab der Heizvorrichtung. Die Funktion des Schlauchadapters mit beispielsweise einem Rückschlagventil oder Druckbegrenzungsventil würde dann, zumindest teilweise, von der okkludierenden Pumpe übernommen werden; diese könnte das Auftreten eines Unterdrucks zwischen Heizvorrichtung und Dialysator durch ihre okkludierende Wirkung verhindern. Eine erfindungsgemäße Anordnung mit einem Dialysierflüssigkeits-Schlauchsystem, in dem die Heizvorrichtung, beispielsweise eine Beutelheizung, stromab der Pumpe angeordnet ist und der Schlauchadapter stromab der Heizvorrichtung angeordnet ist, weist die folgenden Vorteile gegenüber der genannten alternativen Lösung auf: Ein Unterdruck in der Heizvorrichtung ist nicht nur durch den Dialysator verursacht möglich (s. Beschreibung zu Fig. 3: ein hoher TMP im Dialysator kann verschiedene Ursachen haben), sondern auch durch ein Saugen der okkludierenden Pumpe, wie dies bei Rollenpumpen regelmäßig auftritt. Auch durch die Saugwirkung der stromab der Heizvorrichtung angeordneten, okkludierenden Pumpe kann der unerwünschte Effekt des Unterdrucks in der Heizvorrichtung auftreten.

Weiterhin werden mit der erfindungsgemäßen Anordnung mögliche Bilanzierfehler vermieden, da die Heizvorrichtung, vor allem die Beutelheizung, zwar als zusätzlicher Volumenspeicher anzusehen ist, in der Regel aber nicht im Bilanziersystem berücksichtigt ist. Eine Veränderung aufgrund Unterdrucks und Kollabierens des Gefäßes der Heizvorrichtung, vor allem der Beutelheizung, wie dies bei der o. g. alternativen Lösung auftreten kann, kann vorteilhaft mit einem Schlauchadapter des erfindungsgemäßen Dialysierflüssigkeitsschlauchs vermieden werden.

Weiterhin kann erfindungsgemäß vorteilhaft ein Erwärmen der Dialysierflüssigkeit, ohne dass die Dialysierflüssigkeit unmittelbar gefördert wird, wie dies bei einer alternativen Anordnung möglich wäre, bei bestimmten Bicarbonat-haltigen Lösungen vermieden werden. Bei einer derartigen Erwärmung kann die Qualität der Dialysierflüssigkeit beeinträchtigt werden. Es kann zu Ausfällungen der Dialysierflüssigkeit kommen.

Aufgrund der erfindungsgemäßen Anordnung der Pumpe stromauf der Heizvorrichtung kann eine ungewollte Abkühlung der Dialysierflüssigkeit vor dem Eintritt in den Dialysator vorteilhaft vermieden werden. Durch eine ungewollte Abkühlung könnte es zu schwer kalkulierbaren Temperaturabweichungen kommen. Diese können erfindungsgemäß vermieden werden unter Erzielen der hiermit einhergehenden Vorteile.

Zum Erkennen eines Unterdrucks in der Heizvorrichtung, beispielsweise einer Beutelheizung, mit dem Ziel, einen solchen zu vermeiden, bedarf es bei der erfindungsgemäßen Lösung oder der erfindungsgemäßen Anordnung des Schlauchadapters bzw. seiner Komponenten zwischen der Heizvorrichtung und dem Dialysator keiner Überwachung des Filtratdrucks, also des Dialysatflüssigkeitsdrucks im Dialysator unmittelbar an der Membran, oder eines anderen Drucks auf einen geeigneten Mindestwert hin. So liegt etwa bei einer Anordnung zur kontinuierlich veno-venösen Hämodiafiltration (CW-HDF) im Wesentlichen kein relevanter Flusswiderstand zwischen Filtratdrucküberwachung und der Heizvorrichtung vor. Zwar wäre es nach einer Korrektur um einen hydrostatischen Druckunterschied möglich, einen Mindestfiltratdruck zu definieren, bei dem sichergestellt ist, dass der Druck in der Heizvorrichtung ausreichend hoch ist, um ein Kollabieren eines Beutels zu verhindern. Bei Unterschreiten dieses Mindestfiltratdrucks könnte beispielsweise die Flussrate der Pumpe gedrosselt werden. Hierzu müsste allerdings ein Steuer- oder Regelkreis aufgebaut werden, was mit Kosten und Aufwand, ferner mit Wartung, Eichung und dergleichen verbunden ist. Dies ist erfindungsgemäß nicht erforderlich.

Ferner birgt eine solche Lösung die Gefahr, dass es in der Folge zu Fehlalarmen innerhalb des erlaubten Flussratenbereichs der Pumpen kommen könnte und die Verfügbarkeit bestimmter Behandlungsparameter eingeschränkt ist. Es könnte zu nicht interpretierbaren Warnungen (leerlaufender Heizbeutel, Bilanzwarnungen, Heizungsalarme) kommen. Durch den Einsatz des erfindungsgemäßen Schlauchadapters kann vorteilhaft auf eine Überwachung eines Mindestfiltratdrucks verzichtet werden.

Ein weiterer Vorteil besteht darin, dass der Innendruck eines Beutels der Heizvorrichtung mittels des Schlauchadapters des erfindungsgemäßen Dialysierflüssigkeitsschlauchs um einen erforderlichen Wert zwischen 5 und 1000 hPa, bevorzugt zwischen 50 und 400 hPa, besonders bevorzugt zwischen 100 und 350 hPa angehoben werden kann. Dieser Wert wird durch die Auswahl einer handelsüblichen, oben genannten Vorrichtung, wie einem Ventil, anhand des Öffnungsdrucks in Flussrichtung einstellbar oder auswählbar.

Die erfindungsgemäß erzielbare Erhöhung des Drucks wirkt einem unerwünschten Ausgasen der Lösung bei Kontakt mit der heißen Heizoberfläche, wie dies bei niedrigem Druck geschehen kann, vorteilhaft entgegen oder verhindert dies im Wesentlichen. Somit kann eine pH-Verschiebung durch das Ausgasen, vor allem wenn CO₂ ausgegast wird, und somit das unerwünschte Ausfällen von Calciumcarbonat und anderen Verbindungen aus der strömenden Lösung vermieden werden.

Vorteilhaft ist auch, dass der Schlauchadapter als Nachrüstteil auch erst nach Erkennen des Problems einer kollabierenden Heizvorrichtung oder Heizbeutels einsetzbar ist. Damit kann eine teure und aufwendige Integration beispielsweise eines Rückschlagventils oder Druckbegrenzungsventils in jedes einzelne der produzierten Dialysat-Schlauchsysteme vermieden werden.

Die vorliegende Erfindung wird im Folgenden anhand der beigefügten Zeichnung, in welcher identische Bezugszeichen gleiche oder ähnliche Bauteile bezeichnen, exemplarisch erläutert. In den zum Teil stark vereinfachten Figuren gilt:
- **Fig. 1**: zeigt schematisch einen Schlauchadapter eines Dialysierflüssigkeitsschlauchs in einer erfindungsgemäßen Ausführungsform mit einem Rückschlagventil und zwei Schlauchanschlüssen;
- **Fig. 2**: zeigt schematisch ein Dialysierflüssigkeits-Schlauchsystem mit integriertem Rückschlagventil; und
- **Fig. 3**: zeigt schematisch eine erfindungsgemäße medizinische Vorrichtung für die Dialyse mit einem extrakorporalen Blutkreislauf und einem Dialysierflüssigkeits-Schlauchsystem.

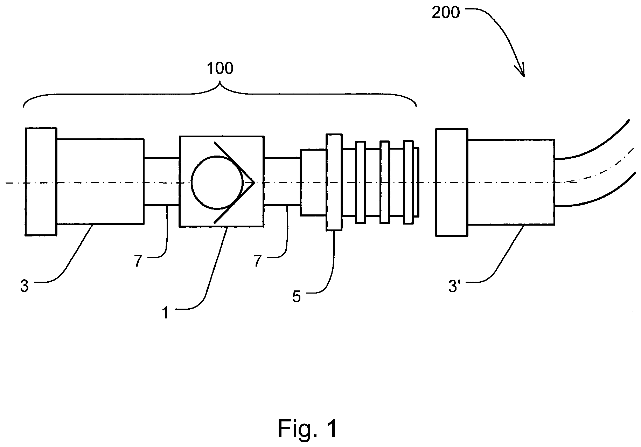

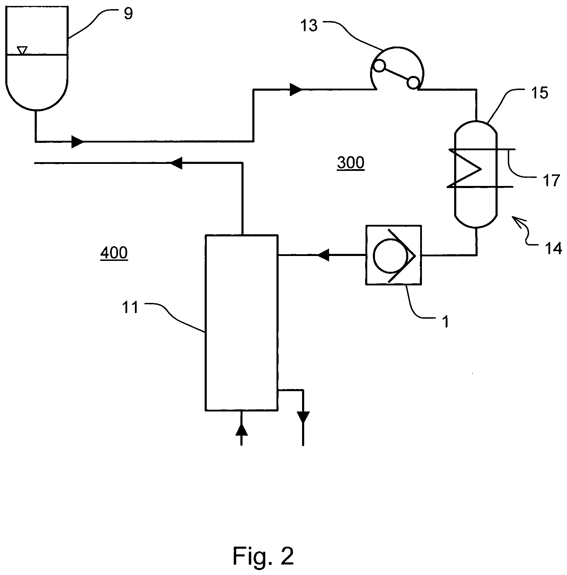

**Fig. 1** zeigt schematisch einen Schlauchadapter 100, der in diesem Ausführungsbeispiel zur Verbindung mit einem Schlauchabschnitt 200 eines Dialysierflüssigkeits-Schlauchsystems 300 (siehe die Fig. 2 und 3) vorgesehen ist.

Der Schlauchadapter 100 besteht in diesem Ausführungsbeispiel aus einem Verschlussmechanismus, der hier exemplarisch als Rückschlagventil 1 (im Folgenden kurz als Ventil 1 bezeichnet) ausgestaltet und als solches symbolisch bezeichnet ist, zwei Schlauchanschlüssen 3, 5 sowie zwei - optional vorgesehenen - kurzen Schlauchabschnitten 7 des Schlauchadapters 100. Der Schlauchadapter 100 kann weitere Komponenten aufweisen.

Die einzelnen Komponenten des Schlauchadapters 100 sind in diesem Ausführungsbeispiel fest miteinander verbunden, beispielsweise mittels Kleben oder Ultraschallverschweißen. Die Komponenten können jedoch auch wieder lösbar miteinander verbunden sein. Letzteres hätte den Vorteil, dass der Schlauchadapter 100 auch noch am Ort seines Gebrauchs mit verschieden großen Schlauchanschlüssen 3, 5 vorgesehen sein kann, welche in oder auf entsprechende Schlauchanschlüsse anderer Schlauchsysteme als dem hier gezeigten Schlauchsystem 300 passen.

Zum Verbinden des Schlauchadapters 100 werden die Schlauchanschlüsse 3, 5 mit Schlauchkupplungen des Dialysierflüssigkeits-Schlauchsystems 300 verbunden. Der Schlauchanschluss 5 ist als erstes Kupplungsteil, hier als sogenanntes "Vaterteil" (auch als Stutzen oder männlicher Verbinder bezeichnet), ausgeführt, der Schlauchanschluss 3 als sogenanntes "Mutterteil" (auch als weiblicher Verbinder bezeichnet). Das Mutterteil der Kupplung (Schlauchanschluss 3) ist in diesem Ausführungsbeispiel baugleich mit einem entsprechenden Schlauchanschluss 3' des bekannten Schlauchabschnitts 200.

Die beiden Schlauchanschlüsse 3', 5 werden als Kupplung zusammengesteckt, so dass das Dialysierflüssigkeits-Schlauchsystem 300 hierdurch um die freiliegende Länge des Schlauchadapters 100 verlängert wird.

Die Strömungsrichtung der Dialysierflüssigkeit durch den Schlauchadapter 100 verläuft - bezogen auf Fig. 1 - bei dessen bestimmungsgemäßen Gebrauch von rechts nach links. Das Ventil 1 des Schlauchadapters 100 öffnet sich - beispielsweise gegen eine Federkraft - dann, wenn stromauf des Ventils 1, also in Fig. 1 rechts davon, ein Mindestdruck anliegt.

Die Funktion des Schlauchadapters 100 bzw. seines Ventils 1 wird anhand von Fig. 2 und 3 näher beschrieben.

**Fig. 2** zeigt das Dialysierflüssigkeits-Schlauchsystem 300 als Teil einer erfindungsgemäßen medizinischen Vorrichtung 600 (siehe Fig. 3), welche hier ein Behandlungssystem für eine Dialyse ist. Die medizinische Vorrichtung 600 weist weiterhin einen Blutkreislauf 400 (hier als Ausschnitt dargestellt, siehe auch hierzu Fig. 3) auf, ferner einen Dialysator 11, der von beiden Fluiden (Blut und Dialysierflüssigkeit) zum Stoffaustausch durchströmt wird.

Das Dialysierflüssigkeits-Schlauchsystem 300, das als Einweg-Schlauchsystem ("Disposable") ausgeführt sein kann, wird von einer Dialysierflüssigkeit aus einem Beutel 9 befüllt. Die Dialysierflüssigkeit wird mittels einer hier nur exemplarisch als Rollenpumpe ausgestalteten Pumpe 13 im Dialysierflüssigkeits-Schlauchsystem 300 gefördert. Dabei kann die Dialysierflüssigkeit aus dem Beutel 9 mittels Schwerkraft und/oder durch Ansaugen mittels der Pumpe 13 in eine zwischen dem Beutel 9 und einer stromab der Pumpe 13, also auf der Druckseite der Pumpe 13, gelegene Heizvorrichtung 14 fließen. Die in den Figuren gezeigte Heizvorrichtung 14 ist exemplarisch als eine Vorrichtung ausgestaltet, welche wenigstens einen Heizbeutel 15 und Heizstäbe oder Heizwendeln 17 aufweist, weshalb sie hierin auch als Beutelheizung bezeichnet wird. Im Heizbeutel 15 wird die Dialysierflüssigkeit erwärmt. Zur Gewährleistung des Wärmeübergangs von der Heizvorrichtung 14 oder den Heizwendeln 17 auf den Heizbeutel 15 ist es hilfreich, ggf. gar notwendig, dass in dem Heizbeutel 15 ein positiver Innendruck der Dialysierflüssigkeit gegenüber dem Umgebungsdruck herrscht. Auf diese Weise legt sich die Beutelwand des Heizbeutels 15 an die Heizwendeln 17 an, was den Wärmeübergang ermöglicht oder begünstigt. Anders ausgedrückt wird mittels eines positiven Innendrucks ein Kollabieren des Heizbeutels 15 und eine Verschlechterung oder Unterbrechung des Wärmeübergangs verhindert.

Weiter stromab der Pumpe 13 und stromab der Heizvorrichtung 14 ist das Ventil 1 angeordnet. In diesem Ausführungsbeispiel ist das Ventil 1 bereits in das Schlauchsystem 300 integriert, Fig. 2 zeigt daher keinen Schlauchadapter 100. Das wie hier integrierte Ventil 1 bedarf daher keiner Schlauchkupplungen, wie sie in Fig. 1 dargestellt sind. Alternativ kann das Ventil 1 jedoch auch als Teil eines Schlauchadapters 100 entsprechend Fig. 1 an das Schlauchsystem 300 angeschlossen werden.

Das Ventil 1 ist derart in dem Schlauchsystem 300 angeordnet, dass bei einem zu niedrigen Druck stromauf des Ventils 1, also zwischen Ventil 1 und Heizvorrichtung 14 oder Heizbeutel 15, das Ventil 1 schließt. Mit diesem Schließen wird auch verhindert, dass sich ein stromab des Ventils 1 herrschender zu niedriger Druck stromaufwärts des Ventils 1 bis in den Heizbeutel 15 fortsetzt und dort möglicherweise zu einem negativen Innendruck (gegenüber dem Umgebungsdruck) oder einem unerwünscht niedrigen Innendruck führt. Wie weiter oben bereits diskutiert wurde, ist ein positiver Innendruck im Heizbeutel 15 hilfreich, um den optimalen, den gewünschten oder den erwarteten Wärmeübergang zu gewährleisten.

Der Öffnungsdruck des Ventils 1 liegt in manchen erfindungsgemäßen Ausführungsformen zwischen 5 und 1000 hPa, bevorzugt zwischen 50 und 400 hPa, besonders bevorzugt zwischen 100 und 350 hPa. Dies bedeutet, dass der Innendruck des Heizbeutels 15 mindestens diesen Wert betragen muss oder mindestens um diesen Wert höher sein muss als ein Druck stromab des Ventils, damit das Ventil 1 öffnet, lässt man die in dem Schlauchabschnitt zwischen Heizbeutel 15 und Ventil 1 auftretenden Strömungsverluste außer Betracht. Anders ausgedrückt muss der Druck stromauf des Ventils 1 mindestens diesen Wert (zwischen 5 und 1000 hPa, bevorzugt zwischen 50 und 400 hPa, besonders bevorzugt zwischen 100 und 350 hPa) betragen oder um diesen Wert höher liegen, um den Öffnungsdruck des Ventils 1 zu überwinden.

Ein niedriger Druck stromab des Ventils 1, und damit ein "Leersaugen" der Dialysierflüssigkeit weiter stromauf bis hin zum Heizbeutel 15, kann in der Praxis verschiedene Ursachen haben. Dies wird mit Blick auf Fig. 3 anhand eines vollständigen Behandlungssystems näher erläutert.

**Fig. 3** zeigt schematisch eine medizinische Vorrichtung 600 (oder auch als Behandlungssystem bezeichnet) für die Dialyse, in diesem erfindungsgemäßen Ausführungsbeispiel speziell für die kontinuierliche veno-venöse Hämodiafiltration (Kombination von Hämofiltration und Hämodialyse), abgekürzt CVV-HDF, mit einem extrakorporalen Blutkreislauf 400, einem Substituat-Schlauchsystem 500 und einem Dialysierflüssigkeits-Schlauchsystem 300.

Dem Patienten wird mittels eines arteriellen Anschlusses 19 einer arteriellen Leitung des extrakorporalen Blutkreislaufs 400 Blut entnommen. Stromab des arteriellen Anschlusses 19 ist ein Absperrhahn 21 angeordnet. Wiederum stromab hiervon wird der arterielle Druck mittels eines Drucksensors 23 gemessen, noch weiter stromab ist eine Blutpumpe 13' angeordnet. Zwischen der Blutpumpe 13' und dem Anschluss der arteriellen Leitung an den Dialysator 11 wird der Hämofiltrationsdruck mittels eines Drucksensors 25 gemessen. Stromab dieses Drucksensors 25 wird dem Blut an einer Zugabestelle 27 Heparin zur Gerinnungshemmung zugegeben.

Im Dialysator 11 findet der Stoffaustausch mit der Dialysierflüssigkeit des Schlauchsystems 300, welches den Dialysator 11 als Dialysat verlässt, statt. Dies wird weiter unten näher beschrieben.

Stromab des Dialysators 11 fließt das Blut in eine venöse Tropfkammer 29, in der mittels eines Drucksensors 31 der venöse Druck gemessen wird. Stromab hiervon ist ein Absperrhahn 33 angeordnet. Das Blut wird mittels eines venösen Anschlusses 35 in das Gefäßsystem des Patienten zurückgeführt.

Das Substituat-Schlauchsystem 500 dient dazu, dem Patienten einen Teil des bei der Behandlung entzogenen Flüssigkeitsvolumens, welches dem Blut durch die Filtration im Dialysator 11 entzogen wurde, zu substituieren. Hierzu wird Substituat-Flüssigkeit aus einem Beutel 9' entnommen. Das Substituat wird im Substituat-Schlauchsystem 500 mittels einer Substituatpumpe 13" in einen Heizbeutel 15' gefördert, dort erwärmt und anschließend dem Blutkreislauf 400 zugeführt.

Das Schlauchsystem 300 stromauf des Dialysators 11 wurde bereits zu Fig. 2 eingehend erläutert. Stromab des Dialysators 11 wird der Filtratdruck mittels eines Drucksensors 37 gemessen, weiter stromab wird das Dialysat gemeinsam mit dem Filtrat mittels einer Pumpe 13‴ in einen Auffangbehälter 39 gefördert oder verworfen.

Nachfolgend werden in der Praxis auftretende Ursachen eines niedrigen Drucks stromab des Ventils 1 diskutiert.

Ein niedriger Druck (gegenüber dem Umgebungsdruck) stromab des Ventils 1, welcher zu einem "Leersaugen" des Heizbeutels 15 führen könnte, wäre nicht das Ventil 1 vorgesehen, kann beispielsweise durch Ablagerungen auf der Filtermembran des Dialysators 11 (auf der Membranseite des Blutkreislauf 400; z. B. durch beginnendes "Clotting" des Blutes) bedingt sein. Dies führt zu einer Verringerung der Permeabilität der Filtermembran im Dialysator und damit zu einem Anstieg des Transmembrandrucks TMP (TransMembrane Pressure).

Unabhängig von diesem Phänomen kann die Verwendung von Filtermembranen mit geringer Permeabilität (es treten die gleichen Effekte wie bei Ablagerungen auf der Membran auf) zu diesem Problem des niedrigen Drucks auf der Dialysatseite des Dialysators 11 führen. Eine geringe Permeabilität führt daher zu einem hohen TMP zum Erreichen eines gewünschten bzw. geforderten Stoffaustauschs im Dialysator 11. Die Verwendung des Schlauchadapters 100 und/oder des Dialysierflüssigkeits-Schlauchsystems 300 kann daher bei Verwendung von Filtermembranen mit geringer Permeabilität vorteilhaft dazu genutzt werden, einen hohen Unterdruck im Heizbeutel 15 zu vermeiden und damit einen optimalen, gewünschten oder den erwarteten Wärmeübergang vom Heizungsgehäuse auf den Heizbeutel 15 zu gewährleisten.

Ein möglicherweise für die Belange des Heizbeutels zu niedriger Druck auf der Dialysatseite tritt besonders bei Dialyse-Behandlungen auf, die mittels der kontinuierlichen veno-venösen Hämodiafiltration (CVV-HDF) durchgeführt werden, was hohe Filtrationsflussraten und ein entsprechend hohes Druckgefälle über die Filtermembran, d.h. einen hohen Transmembrandruck TMP, erfordert.

Der Druckbezugspunkt für den Bereich um die Filtermembran des Dialysators 11 liegt im Bereich des venösen Anschlusses 35. Der Druckbezugspunkt für die Dialysatseite ist der Druckaufnehmer 37. Von dort kann der Druck auf die Dialysatseite der Filtermembran (im Schlauchsystem 300) rückverfolgt werden, indem strömungsbedingte Druckabfälle und hydrostatische Druckunterschiede berücksichtigt werden. Bei hinreichend hohem TMP ist so nachvollziehbar, dass es zu einem Druck im Heizungsbeutel 15 unterhalb des Umgebungsluftdrucks und damit zu einem Kollabieren des Heizbeutels 15 kommen kann, wenn diesem nicht, beispielsweise durch das Verwenden des Schlauchadapters 100 oder des Dialysierflüssigkeits-Schlauchsystems 300, entgegengewirkt wird.

**Bezugszeichenliste**

| **Bezugszeichen** | **Beschreibung** |
|---|---|
| 100 | Schlauchadapter |
| 200 | Schlauchabschnitt |
| 300 | Dialysierflüssigkeits-Schlauchsystem |
| 400 | Blutkreislauf |
| 500 | Substituat-Schlauchsystem |
| 600 | Medizinische Vorrichtung |
| 1 | Rückschlagventil, Ventil |
| 3,3' | Schlauchanschluss, Mutterteil |
| 5 | Schlauchanschluss, Vaterteil |
| 7 | Schlauchabschnitt |
| 9, 9' | Beutel |
| 11 | Dialysator |
| 13 | Dialysierflüssigkeitspumpe |
| 13' | Blutpumpe |
| 13" | Substituatpumpe |
| 13‴ | Dialysatpumpe |
| 14 | Heizvorrichtung |
| 15, 15' | Heizbeutel |
| 17 | Heizwendel, Heizstäbe |
| 19 | arterieller Anschluss |
| 21 | Absperrhahn, arteriell |
| 23 | Drucksensor für arteriellen Druck |
| 25 | Drucksensor für Hämofiltrationsdruck |
| 27 | Zugabestelle für Heparin |
| 29 | venöse Tropfkammer |
| 31 | Drucksensor für venösen Druck |
| 33 | Absperrhahn, venös |
| 35 | venöser Anschluss |
| 37 | Drucksensor für Filtratdruck |
| 39 | Auffangbehälter |

## Patentansprüche

1. Dialysierflüssigkeitsschlauch (300), aufweisend wenigstens
- einen Schlauchadapter (100), verbunden mit wenigstens einem Schlauchabschnitt des Dialysierflüssigkeitsschlauchs (300),
wobei der Schlauchadapter (100) wenigstens einen Verschlussmechanismus zum Verschließen seines durchströmbaren Lumens aufweist,
wobei der Verschlussmechanismus ein Rückschlagventil mit einem vorbestimmten Öffnungsdruck ist,
wobei der Öffnungsdruck des Rückschlagventils mindestens 5 hPa und/oder maximal 1000 hPa beträgt,
- einen Abschnitt, welcher vorgesehen ist, um im Gebrauch mit einer Beutelheizung verbunden zu werden,
- einen Schlauchabschnitt, welcher im Gebrauch in eine Pumpe (13) zum Fördern der Dialysierflüssigkeit innerhalb des Dialysierflüssigkeitsschlauchs (300) einzulegen ist,
wobei der Abschnitt zum Verbinden mit der Beutelheizung stromab des Schlauchabschnitts zum Einlegen in die Pumpe (13) angeordnet ist, und
wobei der Schlauchadapter (100) stromab des Abschnitts zum Verbinden mit der Beutelheizung angeordnet ist.

2. Dialysierflüssigkeitsschlauch (300) nach Anspruch 1, wobei der Öffnungsdruck des Rückschlagventils mindestens 50 hPa und/oder maximal 400 hPa, bevorzugt mindestens 100 hPa und/oder maximal 350 hPa beträgt.

3. Dialysierflüssigkeitsschlauch (300) nach einem der Ansprüche 1 bis 2, wobei der Schlauchadapter integral mit dem Dialysierflüssigkeitsschlauch (300) hergestellt ist.

4. Medizinische Vorrichtung (600), aufweisend wenigstens einen Dialysierflüssigkeitsschlauch (300) nach einem der Ansprüche 1 bis 3 oder hiermit verbunden.

5. Medizinische Vorrichtung (600) nach Anspruch 4, ausgestaltet als Blutreinigungseinrichtung oder Dialysevorrichtung.

## Claims

1. A dialysis fluid tubing (300) comprising at least:
- a tubing adapter (100) connected with at least one tubing section of the dialysis fluid tubing (300),
wherein the tubing adapter (100) comprises at least one lock mechanism for locking its flow-through lumen,
wherein the lock mechanism is a non-return valve with a predetermined opening pressure,
wherein the opening pressure of the non-return valve is at least 5 hPa and/or at most 1000 hPa,
- a section provided for being connected, in use, with a bag heating device,
- a tubing section to be inserted, in use, into a pump (13) for conveying the dialysis fluid within the dialysis fluid tubing (300),
wherein the section to be connected with the bag heating device is arranged downstream of the tubing section intended for insertion into the pump (13), and
wherein the tubing adapter (100) is arranged downstream of the section intended for connection with the bag heating device.

2. The dialysis fluid tubing (300) according to claim 1, wherein the opening pressure of the non-return valve is at least 50 hPa and/or at most 400 hPa, preferably at least 100 hPa and/or at most 350 hPa.

3. The dialysis fluid tubing (300) according to anyone of claims 1 to 2, wherein the tubing adapter is made integral with the dialysis fluid tubing (300).

4. A medical device (600) comprising at least one dialysis fluid tubing (300) according to anyone of claims 1 to 3 or connected thereto.

5. The medical device (600) according to claim 4 designed as a blood purification device or dialysis device.

## Revendications

1. Un tuyau pour liquide de dialyse (300) comprenant au moins:
- un adaptateur de tuyau (100) relié à au moins une section du tuyau pour liquide de dialyse (300),
où l'adaptateur de tuyau (100) comprend au moins un mécanisme d'obturation pour obturer sa lumière traversante,
où le mécanisme d'obturation est une vanne anti-retour avec une pression d'ouverture prédéterminée,
où la pression d'ouverture de la vanne anti-retour est d'au minimum 5 hPa et/ou d'au maximum 1000 hPa,
- une section prévue pour être reliée, en cours d'utilisation, à un dispositif de chauffage à sac,
- une section du tuyau à insérer, en cours d'utilisation, dans une pompe (13) afin d'acheminer le liquide de dialyse à l'intérieur du tuyau pour liquide de dialyse (300),
où la section à relier au dispositif de chauffage à sac est agencée en aval de la section du tuyau à insérer dans la pompe (13), et
où l'adaptateur de tuyau (100) est agencé en aval de la section à relier au dispositif de chauffage à sac.

2. Le tuyau pour liquide de dialyse (300) selon la première revendication, où la pression d'ouverture de la vanne anti-retour est d'au minimum 50 hPa et/ou d'au maximum 400 hPa, de préférence d'au minimum 100 hPa et/ou d'au maximum 350 hPa.

3. Le tuyau pour liquide de dialyse (300) selon l'une quelconque des revendications 1 à 2, où l' adaptateur de tuyau est fabriqué d'un seul tenant avec le tuyau pour liquide de dialyse (300) .

4. Un dispositif médical (600) comprenant au moins un tuyau pour liquide de dialyse (300) selon l'une quelconque des revendications 1 à 3 ou relié à celui-ci.

5. Le dispositif médical (600) selon la revendication 4 conçu comme un dispositif d'épuration du sang ou un appareil de dialyse.
